# EUROPÄISCHE PATENTSCHRIFT

(11) **EP 4 094 076 B1**
(45) Veröffentlichungstag und Bekanntmachung des Hinweises auf die Patenterteilung: **09.04.2025**
(21) Anmeldenummer: 20835741.8
(22) Anmeldetag: 16.12.2020
(51) Int. Cl.: G01N 33/28, G01N 30/88

(54) **VORRICHTUNG UND VERFAHREN ZUR DETEKTION VON GAS**
APPARATUS AND METHOD FOR DETECTING GAS
APPAREIL ET PROCÉDÉ DE DÉTECTION DE GAZ

(30) Priorität: 20.01.2020 DE 102020101132
(43) Veröffentlichungstag der Anmeldung: 30.11.2022
(73) Patentinhaber: Maschinenfabrik Reinhausen GmbH, 93059 Regensburg (DE)
(72) Erfinder: BARSAN, Nicolae, 72074 Tübingen (DE); SCHÜBEL, Jürgen, 61440 Oberursel (DE); KURZ, Andreas, 60437 Frankfurt (DE)
(86) Internationale Anmeldenummer: PCT/EP2020/086393
(87) Internationale Veröffentlichungsnummer: WO 2021/148203

(56) Entgegenhaltungen:
- EP-A1- 2 927 681
- DE-B3- 102013 112 823
- US-B1- 6 490 938
- LEE JUN HO ET AL: "Selective C2H2 detection with high sensitivity using SnO2 nanorod based gas sensors integrated with a gas chromatography", SENSORS AND ACTUATORS B: CHEMICAL, ELSEVIER BV, NL, vol. 307, 23 December 2019 (2019-12-23), XP085992254, ISSN: 0925-4005, [retrieved on 20191223], DOI: 10.1016/J.SNB.2019.127598

## Beschreibung

Die Erfindung betrifft eine Vorrichtung und ein Verfahren zur Detektion von mehreren Gasen in mit Isoliermedium gefüllten Hochspannungsgeräten, insbesondere Hochspannungstransformatoren.

DE 10 2013 112 823 B3 offenbart eine Vorrichtung und ein Verfahren zur Detektion von Gas in mit Isoliermedium gefüllten Hochspannungsgeräten. Die Vorrichtung weist eine Membran auf, welche sich in dem Hochspannungsgerät befindet. An einem Ende ist die Membran über eine Leitung mit einer Messkammer verbunden. In der Messkammer sind sowohl ein Thermoelement, ein Gassensor als auch ein Temperatursensor angeordnet. Ein Trägergas wird mittels einer ersten Pumpe durch die Messkammer und die Membran befördert und mit den Gasen aus dem Isoliermedium angereichert.

Dieses bekannte System ist sehr aufwendig aufgebaut. Die Vielzahl der verwendeten Einzelteile gestaltet das System nicht nur teuer, sondern auch wartungsintensiv. Die Ventile für den Luftaustausch verschleißen besonders schnell und bilden damit eine Schwachstelle im System. Die flächig ausgebildete Membran kann bei plötzlichem Druckanstieg und insbesondere beim Evaluieren des Trägergases besonders schnell brechen.

Aufgabe der Erfindung ist es, eine Vorrichtung zur Detektion von Gasmolekülen in einem mit Isoliermedium gefüllten Hochspannungsgerät bereitzustellen, die kostengünstig, wartungsfreundlich sowie weitestgehend verschleißfrei aufgebaut ist, sowie ein Verfahren zur Detektion von Gasen in mit Flüssigkeiten gefüllten Hochspannungsgeräten anzugeben, welches einen sicheren und genauen Betrieb der Vorrichtung gewährleistet.

Diese Aufgabe wird durch eine Vorrichtung gemäß Anspruch 1 und durch ein Verfahren gemäß Anspruch 5 gelöst. Vorteilhafte Weiterbildungen sind in den Unteransprüchen beschrieben.

Die Erfindung schlägt gemäß einem ersten Aspekt eine Vorrichtung (1) zur Detektion von Gas (4) in einem mit einem Isoliermedium (2) gefüllten Hochspannungsgerät (3), aufweisend:
- einen Einlass (5) zum Einleiten und einen Auslass (6) zum Abführen eines Trägergases (16);
- wenigstens einen Gassensor (12) zur Detektion eines Gases (4);
- eine erste Pumpe (9) zum Befördern des Trägergases (16) in der Vorrichtung (1);
- eine Membran (13), die zumindest aus wenigstens einem semipermeablen Werkstoff besteht, zumindest teilweise von dem Isoliermedium (2) umgeben ist und zumindest teilweise von dem Trägergas (16) angeströmt wird;
- eine zweite Pumpe (10) zum Hineinbefördern des Trägergases (16) in die Vorrichtung (1) und zum Herausbefördern aus der Vorrichtung (1);
   dadurch gekennzeichnet, dass
- eine Trennsäule (19) vorgesehen ist die vor dem Gassensor (12) angeordnet ist und der Gassensor (12) als Sensor-Array ausgebildet ist;
- ein Ventil (20) mit zumindest zwei Betriebszuständen;
- eine Messkammer (11), in der Sensoren für Temperatur, Feuchtigkeit, Druck und der wenigstens eine Gassensor (12) angeordnet sind; wobei
- die Membran (13) einerseits über eine erste Verbindungsleitung (31) mit einem ersten Anschluss (20.1) und andererseits über eine zweite Verbindungsleitung (32) mit einem zweiten Anschluss (20.2) des Ventils (20) verbunden ist;
- die erste Pumpe (9) in der ersten Verbindungsleitung (31) angeordnet ist;
- eine vierte Verbindungsleitung (34) die Trennsäule (19) über einen vierten Anschluss (20.4) des Ventils (20) mit der Messkammer (11) verbindet;
- die Messkammer (11) über eine zweite Leitung (8) mit einem Auslass (6) verbunden ist;
- eine Probenschleife (21) über eine dritte Verbindungsleitung (33) mit einem dritten Anschluss (20.3) und eine vierte Verbindungsleitung (34) mit einem fünften Anschluss (20.5) des Ventils (20) verbunden ist.

Ein Vorteil liegt darin, dass die Erweiterung der Vorrichtung um die Kombination aus einer Trennsäule und einem Sensor-Array einerseits besonders günstig und andererseits besonders genau ist. Sensor-Arrays sind mehrere besonders kompakte Halbleitersensoren die auf einer Einheit angeordnet sind. Dadurch kann eine Messkammer besonders kompakt gestaltet werden. Weiterhin sind Sensor-Arrays im Vergleich zu herkömmlichen Sensoren aus dem Bereich der Gas-in-Öl-Analyse deutlich günstiger. Die Vorschaltung einer Trennsäule vor dem Sensor-Array ermöglicht eine Vortrennung der Gase und verbessert damit die Selektivität der gesamten Vorrichtung. Trennsäulen werden üblicherweise in Laboraufbauten verwendet, jedoch nicht in Vorrichtungen direkt an Hochspannungsgeräten. Weiterhin kann durch die Trennsäule die Anzahl der einzelnen Sensoren im Sensor-Array reduziert werden. Die Messungen werden vereinfacht, was den Rechenaufwand bei der Datenverarbeitung minimiert. Damit kann die zentrale Steuereinrichtung mit den einfachsten Mitteln aufgebaut werden, was sich positiv auf die Kosten der Vorrichtung auswirkt. Diese Kombination führt in Summe zu einer kostengünstigen, robusten, wartungsarmen und weniger fehleranfälligen Vorrichtung. Ein weiterer Vorteil liegt darin, dass nicht das Isoliermedium, sondern das Trägergas umgewälzt wird. Dadurch ist es möglich, vor dem Anreichern und Detektieren frisches Trägergas wie, z.B. Umgebungsluft, in die Vorrichtung einzuleiten und nach der Detektion aus der Vorrichtung abzuführen.

Das Sensor-Array kann nach Bedarf auf beliebige Art und Weise ausgebildet sein, beispielsweise als Halbleitersensor bestehend aus Oxiden der Metalle oder Übergangsmetalle wie z.B. Zinndioxid (SnO2), Wolframoxid (WO3) und/oder mit Edelmetallen, wie z.B. Platin (Pt) oder Palladium (Pd), dotiert. Das Sensor-Array kann beispielsweise auf Basis des Pellistor-Prinzips aufgebaut sein und/oder ein Taguchi-Gassensor sein.

Die Trennsäule kann nach Bedarf auf beliebige Art und Weise ausgebildet sein, beispielsweise entweder aus Metall oder aus Quarzglas. Sie kann innen mit einer definierten stationären Phase ausgekleidet sein, z.B. mit zähflüssigen Polyorganosiloxanen. Die Länge der Trennsäule beträgt vorzugsweis ca. 0,5 bis 1,0m. Die Trennsäule kann vom Säulentyp PoraBond Q, WP Plot Q, Hayesep N oder Hayesep Q sein.

Das Hochspannungsgerät kann nach Bedarf auf beliebige Art und Weise ausgebildet sein, beispielsweise als Hochspannungstransformator, Leistungstransformator, Laststufenschalter, Leistungsschalter, Kondensatordurchführung oder ein anderes ölgefülltes elektrisches Betriebsmittel.

Das Isoliermedium kann nach Bedarf auf beliebige Art und Weise ausgebildet sein, beispielsweise als Isolieröl bzw. Esterflüssigkeit.

Das zu detektierende Gas kann nach Bedarf auf beliebige Art und Weise ausgebildet sein und beispielsweise wenigstens eine Kohlenwasserstoffverbindung und/oder andere Gasmoleküle und/oder andere Gasatome enthalten.

Die semipermeable Membran kann nach Bedarf auf beliebige Art und Weise ausgebildet sein und beispielsweise zumindest teilweise aus Teflon bestehen.

Die Pumpen können nach Bedarf auf beliebige Art und Weise ausgebildet sein, beispielsweise als Membranpumpen.

Es kann vorgesehen sein, dass
- ein Ventil mit zumindest zwei Betriebszuständen vorgesehen ist.

Das Ventil kann nach Bedarf auf beliebige Art und Weise ausgebildet sein, beispielsweise als Valco 6-Port Ventil oder auch als Valco 8-Port Ventil. Das Ventil kann vorzugsweise in mindestens zwei Betriebszustände geschaltet werden.

Es kann vorgesehen sein, dass
- ein Einlass eine erste Leitung und einen Filter vorgesehen sind und die erste Leitung mit einem sechsten Anschluss des Ventils verbunden ist;
- die Membran einerseits über eine erste Verbindungsleitung mit einem ersten Anschluss und andererseits über eine zweite Verbindungsleitung mit einem zweiten Anschluss des Ventils verbunden ist;
- die erste Pumpe in der ersten Verbindungsleitung angeordnet ist;
- eine Messkammer vorgesehen ist, in der Sensoren für Temperatur, Feuchtigkeit und Druck und /oder Gassensoren angeordnet sind;
- eine vierte Verbindungsleitung die Trennsäule über einen vierten Anschluss des Ventils mit der Messkammer verbindet;
- die Messkammer über eine zweite Leitung mit einem Auslass verbunden ist.

Es kann vorgesehen sein, dass
- eine Probenschleife über eine dritte Verbindungsleitung mit einem dritten Anschluss und eine vierte Verbindungsleitung mit einem fünften Anschluss des Ventils verbunden ist.

Die Probenschleife kann nach Bedarf auf beliebige Art und Weise ausgebildet sein und beispielsweise zumindest teilweise aus Teflon bestehen.

Es kann vorgesehen sein, dass
- die Sensoren in der Messkammer, die erste Pumpe, die zweite Pumpe und das Ventil mit einer Steuereinrichtung verbunden sind; und
- die Sensoren in der Messkammer, die erste Pumpe, die zweite Pumpe und das Ventil anhand der Messungen der Sensoren in der Messkammer gesteuert werden.

Es kann vorgesehen sein, dass
- der Einlass eine erste Leitung, eine Pumpe und einen Filter aufweist.

Es kann vorgesehen sein, dass
- die Membran zumindest teilweise spiralförmig und/oder zumindest teilweise mäanderförmig und/oder zumindest teilweise wendelförmig ausgebildet ist.

Es kann vorgesehen sein, dass
- wenigstens ein Temperatursensor vorgesehen ist.

Es kann vorgesehen sein, dass
- der Auslass eine zweite Leitung aufweist.

Es kann vorgesehen sein, dass
- eine Leitung oder Verbindungsleitung für den Transport der zu analysierenden Gase vorhanden ist, die eine Pumpe aufweist.

Es kann vorgesehen sein, dass
- eine Messkammer vorgesehen ist, in der Sensoren zur Bestimmung von Temperatur, Umgebungsfeuchtigkeit, Druck und von verschiedenen Gasen angeordnet sind.

Es kann vorgesehen sein, dass
- in der Messkammer wenigstens ein Thermoelement angeordnet ist.

Es kann vorgesehen sein, dass
- der Temperatursensor und das Thermoelement mit einer Steuereinrichtung verbunden sind und
- das Thermoelement anhand der Messungen des Temperatursensors gesteuert wird.

Es kann vorgesehen sein, dass
- während des Betriebes der ersten Pumpe die zweite Pumpe die Funktion eines verschlossenen Ventils hat und während des Betriebes der zweiten Pumpe die erste Pumpe die Funktion eines verschlossenen Ventils hat.

Es kann vorgesehen sein, dass
- das Thermoelement als Peltier-Element ausgebildet ist.

Es kann vorgesehen sein, dass
- die Messkammer im Inneren mit einem inerten Material, Platin oder Gold oder einem anderen Edelmetall ausgekleidet ist.

Die Erfindung schlägt gemäß einem zweiten Aspekt ein Verfahren zur Detektion von mehreren Gasen in einem mit einem Isoliermedium gefüllten Hochspannungsgerät vor, vorzugsweise mittels einer Vorrichtung nach einem der vorherigen Ansprüche, wobei:
- in einem ersten Schritt eine Messkammer mit einem Trägergas gespült wird, indem das Trägergas durch die Trennsäule in die Messkammer hinein und aus der Messkammer heraus befördert wird;
- in einem zweiten Schritt das Trägergas durch eine Membran hindurch befördert wird und sich durch Gas anreichert, das die Membran durchströmt;
- in einem dritten Schritt ein Sensor in der Messkammer das Gas, nach dem dieses die Trennsäule durchquert hat, misst.

Es kann vorgesehen sein, dass
- im ersten und dritten Schritt das Ventil in einem ersten Betriebszustand zum Spülen der Messkammer und zum Messen der Gase ist;
- im dritten Schritt das Ventil in einem zweiten Betriebszustand zum Anreichern des Trägergase ist.

Es kann vorgesehen sein, dass
- das Trägergas durch ein Ventil, eine Probenschleife, die vierte Verbindungsleitung und die Trennsäule in die Messkammer transportiert wird und dort von den Sensoren die in der Membran gesammelten Gase detektiert werden.

Es kann vorgesehen sein, dass
- beim Spülen das Trägergas durch eine erste Leitung angesaugt, durch das Ventil und über die Trennsäule in die Messkammer befördert und durch den Auslass abgeführt wird.

Es kann vorgesehen sein, dass
- das Spülen der Messkammer mittels der zweiten Pumpe erfolgt.

Es kann vorgesehen sein, dass
- das Befördern des Trägergases mittels einer ersten Pumpe erfolgt.

Es kann vorgesehen sein, dass
- die Menge und/oder Art des Gases in der Messkammer bestimmt werden, bevor und/oder nachdem das Trägergas heraus befördert wurde.

Es kann vorgesehen sein, dass
- die Menge und/oder Art des Gases in der Messkammer bestimmt werden, bevor und/oder nachdem das Trägergas befördert wurde.

Nachfolgend sind die Erfindung und ihre Vorteile unter Bezugnahme auf die beigefügten Zeichnungen ausführlicher beschrieben. Es zeigen:
- Fig. 1: eine erste Ausführungsform einer Vorrichtung zur Detektion von Gas;
- Fig. 2: einen ersten Betriebszustand der ersten Ausführungsform einer Vorrichtung zur Detektion von Gas;
- Fig. 3: einen zweiten Betriebszustand der ersten Ausführungsform einer Vorrichtung zur Detektion von Gas;
- Fig. 4: eine zweite Ausführungsform einer Vorrichtung zur Detektion von Gas;
- Fig. 5: einen ersten Betriebszustand der zweiten Ausführungsform einer Vorrichtung zur Detektion von Gas;
- Fig. 6: einen zweiten Betriebszustand der zweiten Ausführungsform einer Vorrichtung zur Detektion von Gas;
- Fig. 7: ein Verfahren zur Detektion von Gas.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dafür dar, wie der erfindungsgemäße Vorrichtung ausgebildet sein kann; und stellen somit keine abschließende Begrenzung der Erfindung dar.

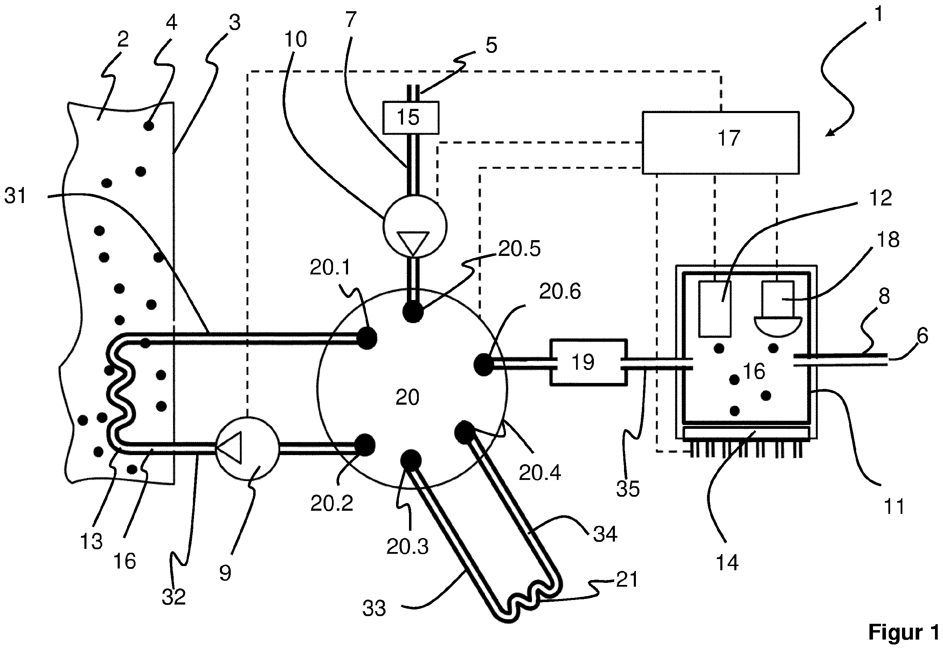

**Figur 1** zeigt eine erste Ausführungsform einer Vorrichtung 1 zur Detektion von Gasmolekülen, Ionen oder Gasen 4 in einem Hochspannungsgerät 3 schematisch dargestellt, welches mit einer Flüssigkeit oder einem Isoliermedium 2 gefüllt ist. Das Hochspannungsgerät 3 kann als Hochspannungstransformator, Leistungstransformator, Laststufenschalter, Leistungsschalter oder Kondensatordurchführung ausgebildet sein.

Die Vorrichtung 1 weist eine Membran oder Kapillare 13 auf, die aus mindestens einem semipermeablen Werkstoff besteht und rohrartig oder schlauchartig aufgebaut ist. Die rohrartige Membran 13 kann beliebig ausgeformt sein, beispielsweise spiralförmig und/oder wendelförmig und/oder mäanderförmig. Durch diese vorteilhafte Ausgestaltung der Membran 13 ist diese für besonders hohe Drücke geeignet. Die Membran 13 befindet sich im Hochspannungsgerät 3 oder zumindest in einem Teil des Hochspannungsgerätes, dem das Isoliermedium 2 zugänglich ist. Somit kann die Membran 13 in einem Buchholzrelais, einer Leitung der Kühlung, etc. angeordnet sein. Durch die rohrartige Ausgestaltung und den in eine Richtung gasdurchlässigen (semipermeablen) Werkstoff können Moleküle des Gases 4 in einen Kreislauf der Vorrichtung 1 gelangen.

Die Membran 13 ist an einem Ende, das ihren Eingang bildet, über eine erste Verbindungsleitung 31 mit einen ersten Anschluss 20.1 eines Ventils 20 und andererseits an einem anderen Ende, das ihren Ausgang bildet, über eine zweite Verbindungsleitung 32 mit dem zweiten Anschluss 20.2 des Ventils 20 verbunden. In der zweiten Leitung 32 ist eine erste Pumpe 9 angeordnet.

Weiterhin weist die Vorrichtung eine Probenschleife 21 auf. Eine Probenschleife ist eine gasdichte Kapillare mit einem definierten Volumen. Diese ist meist aus fused silica hergestellt. Sie kann jedoch auch aus einem geeigneten Kunststoff bestehen. Die Probenschleife 21 ist über eine dritte und eine vierte Verbindungsleitung 33, 34 mit dem dritten und vierten Anschluss 20.3, 20.4 des Ventils 20 verbunden. Die Vorrichtung 1 weist weiterhin einen erste Leitung 7 auf, die einerseits einen Einlass 5 hat und auf der anderen Seite mit einem fünften Anschluss 20.5 des Ventils 20 verbunden ist. In der ersten Leitung 7 sind ein Filter 15 und eine zweite Pumpe 10 angeordnet.

Zwischen dem sechsten Anschluss 20.6 und der Messkammer 11 ist eine Trennsäule 19 über eine fünfte Verbindungsleitung 35 angeordnet. Die Messkammer 11 ist außerdem über eine zweite Leitung 8 mit einem Auslass 6 verbunden.

Die Messkammer 11 weist ein Thermoelement 14 bspw. Peltier-Element auf, mit dessen Hilfe eine Temperierung der Messkammer 11 durchgeführt wird. Zusätzlich sind in der Messkammer 11 mindestens ein Gassensor 12 und ein Temperatursensor 18 angeordnet.

Der Gassensor 12 ist als Sensor-Array ausgestaltet. Dieses Sensor-Array besteht aus mehreren Halbleitersensoren. Die Halbleitersensoren bestehen aus Oxiden der Metalle oder Übergangsmetalle wie z.B. Zinndioxid (SnO2), Wolframoxid (WO3) und sind zur Verbesserung der Selektivität mit Edelmetallen wie z.B. Platin (Pt) oder Palladium (Pd) dotiert. Das Sensor-Array wird auf Basis des Pellistor-Prinzips aufgebaut, dies stellt jedoch nicht die einzige Bauweise dar. Klassische Halbleitersensoren sind Taguchi-Gassensoren.

Die Messkammer 11 ist im Inneren mit einem inerten Material, wie z.B. Gold, ausgekleidet bzw. beschichtet. Diese Beschichtung bietet den Vorteil, dass sich die Gase 4 nicht im Inneren niederschlagen bzw. kondensieren und nichtreproduzierbar anlagern, dabei zumindest eine polare, physikalische Bindung eingehen und damit in der Gesamtgasbilanz fehlen können, womit falsche Werte im Vergleich zu einer Laboranalyse gemessen würden.

Die Trennsäule 19 kann entweder aus Metall oder aus Quarzglas bestehen. Sie ist innen mit einer definierten stationären Phase ausgekleidet z.B. mit zähflüssigen Polyorganosiloxanen. Die Länge der Trennsäule beträgt ca. 0,5 - 1,0m. Gängige Säulentypen sind PoraBond Q, WP Plot Q, Hayesep N oder Hayesep Q.

Das Ventil 20 weist unterschiedliche Betriebszustände/Stellungen auf. Bei diesen werden die einzelnen Anschlüsse intern derart beschaltet bzw. verbunden, dass unterschiedliche Kreisläufe bzw. Verbindungen einzelner Komponenten untereinander in der Vorrichtung 1 entstehen.

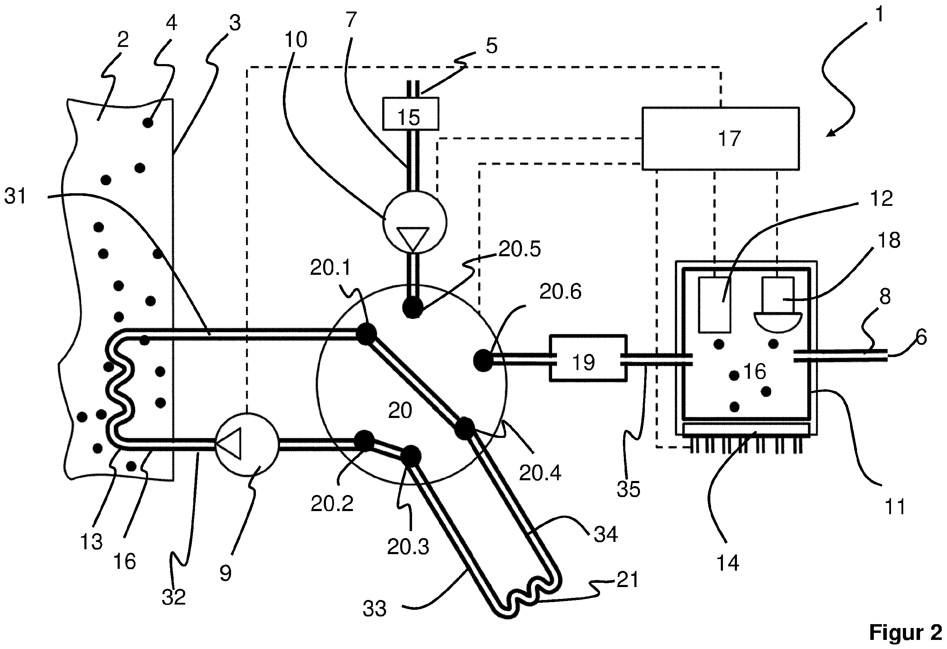

In einem ersten Betriebszustand, wie in **Figur 2** dargestellt ist, wird beispielsweise ein Kreislauf zur Anreichern des Trägergases 16 erzeugt. Hier werden der erste und der vierte Anschluss 20.1, 20.4 und der zweite Anschluss mit dem dritten Anschluss 20.2 und 20.3 intern verbunden. Die erste Pumpe 9 befördert das Trägergas 16 durch die zweite Verbindungsleitung 32, die Kapillare 13 und die Probenschleife 21. Da der Druck im Inneren des Hochspannungsgerätes 3 stets höher ist als der Druck der Umgebung und somit auch der Druck in der Vorrichtung 1, gelangen die im Isoliermedium 2 gelösten Gase 4 in den Kreislauf der Vorrichtung 1 durch die semipermeable Membran 13. Das Trägergas 16 wird durch wiederholte Beförderung bzw. Umwälzung angereichert. Das Ventil 20 ist ebenfalls mit der zentralen Steuereinrichtung 17 verbunden. Als Trägergas 16 kann Luft aus der Umgebung verwendet werden.

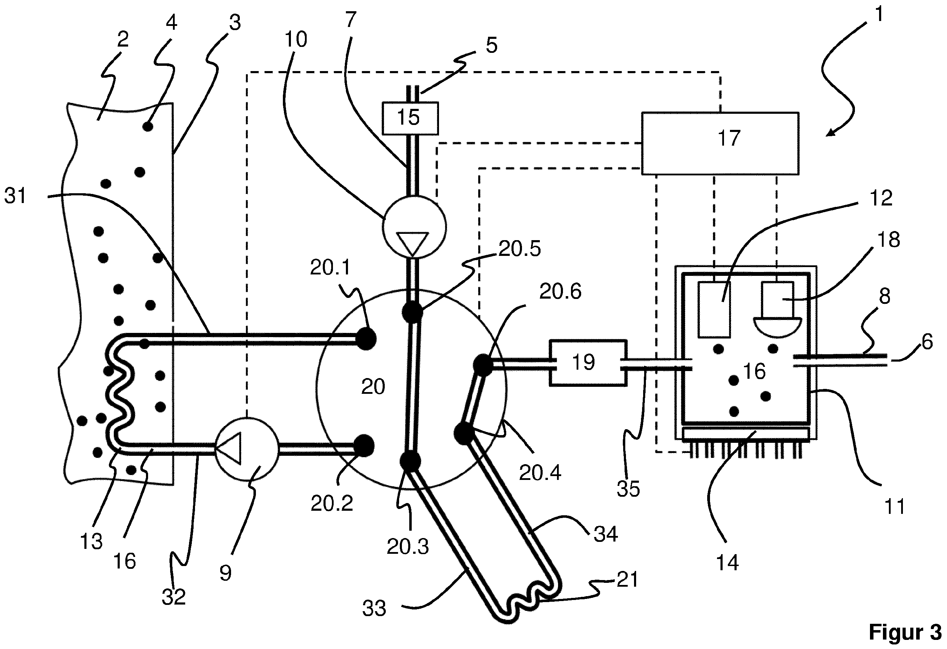

In **Figur 3** wird ein zweiter Betriebszustand des Ventils 20 dargestellt. Hier sind der fünfte und der dritte Anschluss 20.5, 20.3 sowie der vierte und der sechste Anschluss 20.4, 20,6 des Ventils 20 intern miteinander verbunden. Frisches Trägergas 16 (z.B. Umgebungsluft) wird über den Einlass 5 der ersten Leitung 7 mittels der zweiten Pumpe 10 in die Vorrichtung 1 befördert. Das sich in der Probeschleife 21 befindende Trägergas 16 wird zunächst in die Trennsäule 19 und anschließend in die Messkammer 12 eingebracht. Anschließend wird das Trägergas 16 und die angesaugte Luft durch die zweite Leitung 8 zum Auslass 6 befördert. Der Gassensor 12, der Temperatursensor 18 und das Thermoelement 14 in der Messkammer 11 sowie die erste und die zweite Pumpe 9, 10 sind mit einer zentralen Steuereinrichtung 17 verbunden. Die Steuerung des Thermoelementes 14 erfolgt anhand der Messungen des Temperatursensors 18.

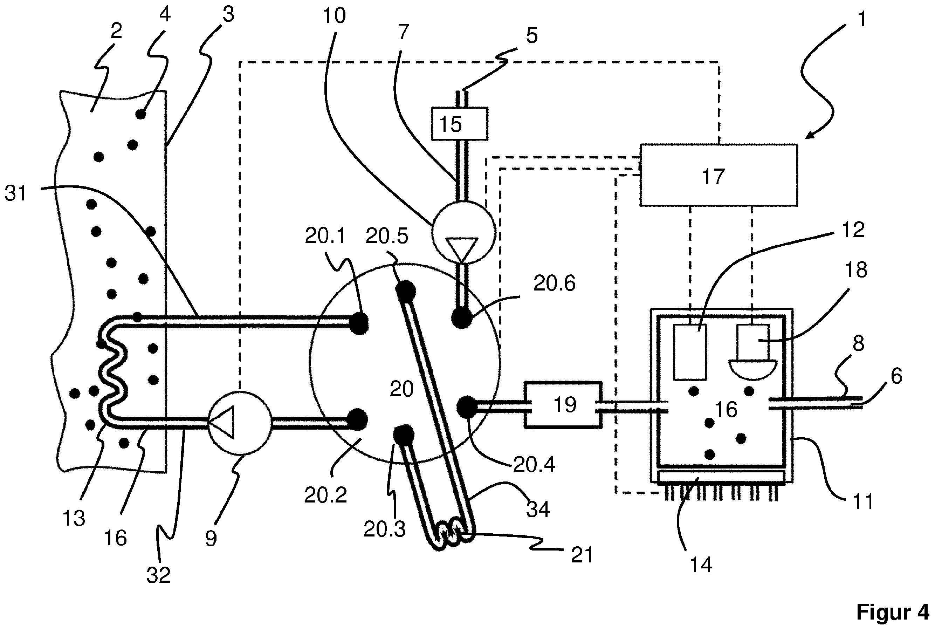

Das Ventil 20 kann vorzugsweise als Valco 6-Port Ventil oder auch als Valco 8-Port Ventil ausgestaltet sein. Dieses Ventil kann intern zwei Betriebszustände annehmen. Bei der Verwendung eines Velco 6-Port Ventils sind die einzelnen Teile der Vorrichtung 1 auf eine bestimmte Weise mit dem Ventil 20 verbunden. Dies ist in **Figur 4** dargestellt. Es ist dabei immer nur möglich jeweils zwei benachbarte Anschlüsse des Ventils intern miteinander zu verbinden. Dadurch ist das Ventil besonders günstig und einfach zu betätigen. Die Membran 13 ist an einem Ende, das ihren Eingang bildet, über eine erste Verbindungsleitung 31 mit einen ersten Anschluss 20.1 eines Ventils 20 und andererseits an einem anderen Ende, das ihren Ausgang bildet, über eine zweite Verbindungsleitung 32 mit dem zweiten Anschluss 20.2 des Ventils 20 verbunden. In der zweiten Leitung 32 ist eine erste Pumpe 9 angeordnet.

Weiterhin weist die Vorrichtung eine Probenschleife 21 auf. Die Probenschleife 21 ist über eine dritte und eine vierte Verbindungsleitung 33, 34 mit dem dritten und fünften Anschluss 20.3, 20.5 des Ventils 20 verbunden. Die Vorrichtung 1 weist weiterhin einen erste Leitung 7 auf, die einerseits einen Einlass 5 hat und auf der anderen Seite mit einem sechsten Anschluss 20.6 mit dem Ventil 20 verbunden ist. In der ersten Leitung 7 sind ein Filter 15 und eine zweite Pumpe 10 angeordnet.

Zwischen dem vierten Anschluss 20.4 und der Messkammer 11 ist eine Trennsäule 19 über eine fünfte Verbindungsleitung 35 angeordnet. Die Messkammer 11 ist außerdem über eine zweite Leitung 8 mit einem Auslass 6 verbunden.

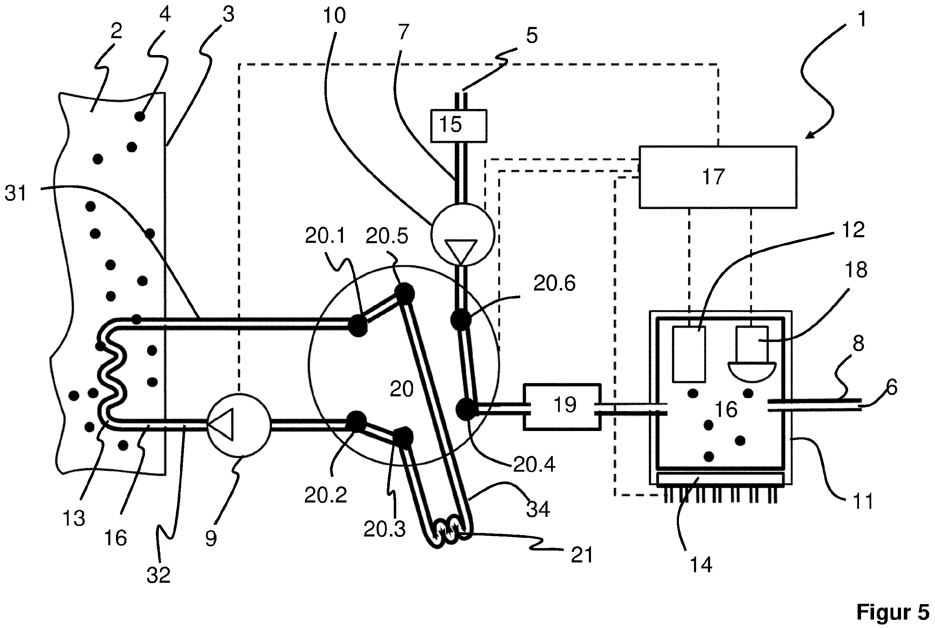

In einem ersten Betriebszustand, wie in **Figur 5** dargestellt ist, wird beispielsweise ein Kreislauf zum Anreichern bzw. Anreicherung des Trägergases 16 erzeugt. Hier werden der erste und der fünfte Anschluss 20.1, 20.5 und der zweite Anschluss mit dem dritten Anschluss 20.2, 20.3 intern verbunden. Der fünfte und der vierte Anschluss 20.4, 20.5 sind ebenfalls miteinander verbunden. Dies ist für die Anreicherung unerheblich. Die erste Pumpe 9 befördert das Trägergas 16 durch die zweite Verbindungsleitung 32, die Kapillare 13 und die Probenschleife 21. Da der Druck im Inneren des Hochspannungsgerätes 3 stets höher ist als der Druck der Umgebung und somit auch der Druck in der Vorrichtung 1, gelangen die im Isoliermedium 2 gelösten Gase 4 in den Kreislauf der Vorrichtung 1 durch die semipermeable Membran 13. Das Trägergas 16 wird durch wiederholte Beförderung bzw. Umwälzung angereichert. Das Ventil 20 ist ebenfalls mit der zentralen Steuereinrichtung 17 verbunden. Als Trägergas 16 kann Luft aus der Umgebung verwendet werden.

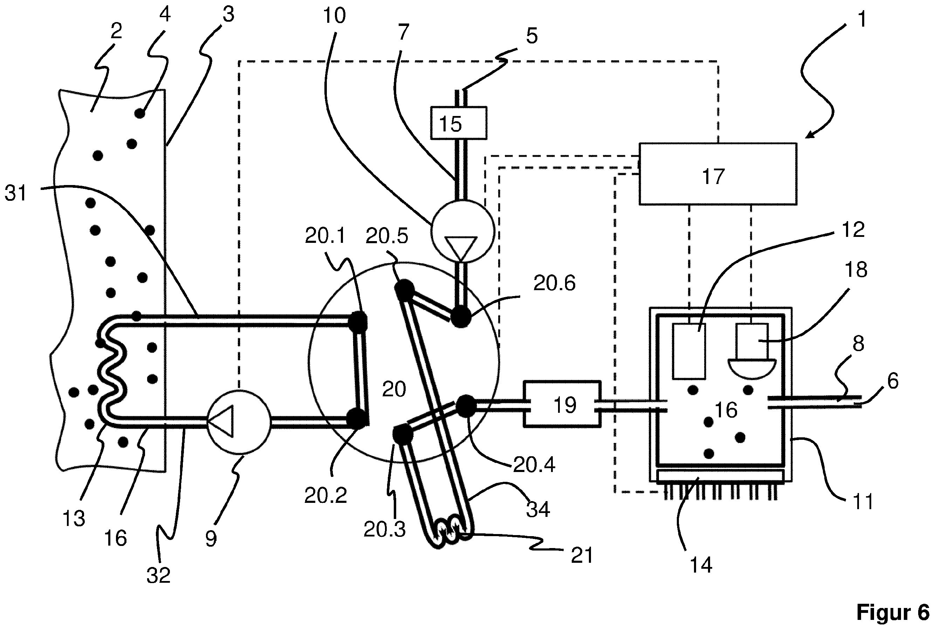

In **Figur 6** wird ein zweiter Betriebszustand des Ventils 20 dargestellt. Hier sind der dritte und der vierte Anschluss 20.3, 20.4 sowie der fünfte und der sechste Anschluss 20.5, 20,6 des Ventils 20 intern miteinander verbunden. Frisches Trägergas 16 (z.B. Umgebungsluft) wird über den Einlass 5 der ersten Leitung 7 mittels der zweiten Pumpe 10 in die Vorrichtung 1 befördert. Das sich in der Probeschleife befindende Trägergas 16 wird zunächst in die Trennsäule 19 und anschließend in die Messkammer 12 eingebracht. Anschließend wird das Trägergas 16 bzw. die angesaugte Luft durch die zweite Leitung 8 zum Auslass 6 befördert.

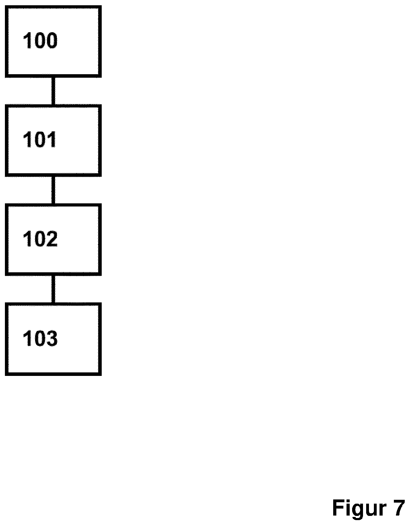

**Figur 7** zeigt ein Ablaufdiagramm für ein Verfahren zur Detektion von Gasen in einem, mit einer Flüssigkeit 2 gefüllten Hochspannungsgerät 3, mittels der Vorrichtung 1 mit folgenden Schritten:
Schritt 100: Zunächst wird die Vorrichtung 1 gespült. Das Ventil 20 ist dabei im zweiten Betriebszustand. Die erste Pumpe 9 ist ausgeschaltet, wohingegen die zweite Pumpe 10 eingeschaltet ist. Über den Einlass 5, also den Filter 15 und die erste Leitung 7, wird das Trägergas 16, z.B. Umgebungsluft, angesaugt. Dabei durchquert das Trägergas 16, den Filter 15, das Ventil 20, die Probenschleife 21, die Trennsäule 19 und die Messkammer 11 bis es zum Auslass 6 gelangt. Die Vorrichtung 1 wird so gespült.
Schritt 101: Nach einer vorgegebenen Zeit oder anhand der Messungen der Gassensoren 12 in der Messkammer 11 wird das Spülen beendet. Die zum Schluss in der Messkammer 11 ermittelten Parameter dienen als Startpunkt oder Nullpunkt für die weiteren Messungen.
Schritt 102: In der Anreicherungsphase der Ausführungsform der Figur 3 und 6 wird das Ventil 20 in den ersten Betriebszustand geschaltet und die die erste Pumpe 9 eingeschaltet, wodurch das Trägergas 16 in der Vorrichtung 1 befördert bzw. umgewälzt wird. Die zweite Pumpe 10 ist ausgeschaltet. Das Trägergas 16 wird in einem Kreislauf zwischen der Probenschleife 21 und der Membran 13 bewegt. Da der Druck im Inneren des Hochspannungsgerätes 3 größer ist als der Druck in der Vorrichtung 1, gelangen Gase 4 aus dem Isoliermedium 2 durch die in eine Richtung gasmoleküldurchlässige Membran 13 in die Vorrichtung 1. Damit kommt es zu einer Anreicherung des Trägergases 16. Die Dauer der Anreicherung kann variabel gestaltet werden.
Schritt 103: Nach der Anreicherungsphase erfolgt die Umschaltung des Ventils 20 in den zweiten Betriebszustand. Das angereicherte Gas in der Probenschleife 21 wird mit dem Trägergas 16 durch die Trennsäule 19 in die Messkammer 11 transportiert und die Menge und die Art der Gase 4 in der Messkammer 11 über den Gassensor 12 bestimmt. Nach der Bestimmung der Gase 4 wird die Vorrichtung 1 gespült, indem frische Umgebungsluft weiter angesaugt wird.

Das beschriebene Verfahren kann entweder permanent oder aber einige Male pro Tag durchgeführt werden. Ein diskontinuierlicher Betrieb der Vorrichtung 1 kann zu einer Erhöhung der Lebenszeit der in der Messkammer 11 verwendeten Gassensoren 12 führen.

### Bezugszeichenliste

- 1: Vorrichtung
- 2: Isoliermedium
- 3: Hochspannungsgerät
- 4: Gas
- 5: Einlass
- 6: Auslass
- 7: erste Leitung
- 8: zweite Leitung
- 9: erste Pumpe
- 10: zweite Pumpe
- 11: Messkammer
- 12: Gassensor
- 13: Membran
- 14: Thermoelement
- 15: Filter
- 16: Trägergas
- 17: Steuereinrichtung
- 18: Temperatursensor
- 19: Trennsäule
- 20: Verteiler/Ventil/Umschaltventil
- 20.1: erster Anschluss
- 20.2: zweiter Anschluss
- 20.3: dritter Anschluss
- 20.4: vierter Anschluss
- 20.5: fünfter Anschluss
- 20.6: sechster Anschluss
- 21: Probenschleife
- 31: erste Verbindungsleitung
- 32: zweite Verbindungsleitung
- 33: dritte Verbindungsleitung
- 34: vierte Verbindungsleitung
- 35: fünfte Verbindungsleitung

## Patentansprüche

1. Vorrichtung (1) zur Detektion von Gas (4) in einem mit einem Isoliermedium (2) gefüllten Hochspannungsgerät (3), aufweisend:
- einen Einlass (5) zum Einleiten und einen Auslass (6) zum Abführen eines Trägergases (16);
- wenigstens einen Gassensor (12) zur Detektion eines Gases (4);
- eine erste Pumpe (9) zum Befördern des Trägergases (16) in der Vorrichtung (1);
- eine Membran (13), die zumindest aus wenigstens einem semipermeablen Werkstoff besteht, zumindest teilweise von dem Isoliermedium (2) umgeben ist und zumindest teilweise von dem Trägergas (16) angeströmt wird;
- eine zweite Pumpe (10) zum Hineinbefördern des Trägergases (16) in die Vorrichtung (1) und zum Herausbefördern aus der Vorrichtung (1);
**dadurch gekennzeichnet, dass**
- eine Trennsäule (19) vorgesehen ist die vor dem Gassensor (12) angeordnet ist und der Gassensor (12) als Sensor-Array ausgebildet ist;
- ein Ventil (20) mit zumindest zwei Betriebszuständen;
- eine Messkammer (11), in der Sensoren für Temperatur, Feuchtigkeit, Druck und der wenigstens eine Gassensor (12) angeordnet sind;
wobei
- die Membran (13) einerseits über eine erste Verbindungsleitung (31) mit einem ersten Anschluss (20.1) und andererseits über eine zweite Verbindungsleitung (32) mit einem zweiten Anschluss (20.2) des Ventils (20) verbunden ist;
- die erste Pumpe (9) in der ersten Verbindungsleitung (31) angeordnet ist;
- eine vierte Verbindungsleitung (34) die Trennsäule (19) über einen vierten Anschluss (20.4) des Ventils (20) mit der Messkammer (11) verbindet;
- die Messkammer (11) über eine zweite Leitung (8) mit einem Auslass (6) verbunden ist;
- eine Probenschleife (21) über eine dritte Verbindungsleitung (33) mit einem dritten Anschluss (20.3) und eine vierte Verbindungsleitung (34) mit einem fünften Anschluss (20.5) des Ventils (20) verbunden ist.

2. Vorrichtung nach Anspruch 1, wobei
- die Membran (13) zumindest teilweise rohrartig und/oder zumindest teilweise schlauchartig ausgebildet ist.

3. Vorrichtung nach einem der Ansprüche 1 bis 2, wobei
- ein Einlass (5), eine erste Leitung (7) und ein Filter (15) vorgesehen sind und die erste Leitung (7) mit einem sechsten Anschluss (20.6) des Ventils (20) verbunden ist.

4. Vorrichtung nach einem der Ansprüche 1 bis 3, wobei
- die Sensoren in der Messkammer (11), die erste Pumpe (9), die zweite Pumpe (10) und das Ventil (20) mit einer Steuereinrichtung (17) verbunden sind; und
- die Sensoren in der Messkammer (11), die erste Pumpe (9), die zweite Pumpe (10) und das Ventil (20) anhand der Messungen des der Sensoren in der Messkammer (11) gesteuert werden.

5. Verfahren zur Detektion von Gas (4) in einem mit einem Isoliermedium (2) gefüllten Hochspannungsgerät (3) mittels einer Vorrichtung (1) nach einem der vorherigen Ansprüche, wobei:
- in einem ersten Schritt eine Messkammer (11) mit einem Trägergas (16) gespült wird, indem das Trägergas (16) durch die Trennsäule (19) in die Messkammer (11) hinein und aus der Messkammer (11) heraus befördert wird;
- in einem zweiten Schritt das Trägergas (16) durch eine Membran (13) hindurch befördert wird und sich durch Gas (4) anreichert, das die Membran (13) durchströmt;
- in einem dritten Schritt ein Sensor (12) in der Messkammer (11) das Gas (4), nach dem dieses die Trennsäule (19) durchquert hat, misst.

6. Verfahren nach Anspruch 5, wobei
- im ersten und dritten Schritt das Ventil (20) in einen ersten Betriebszustand zum Spülen der Messkammer (11) und zum Messen der Gase (4) ist;
- im dritten Schritt das Ventil (20) in einem zweiten Betriebszustand zum Anreichern des Trägergase (16) ist.

7. Verfahren nach Anspruch 5 oder 6, wobei
- das Trägergas (16) durch ein Ventil (20), eine Probenschleife (21), die vierte Verbindungsleitung (34) und die Trennsäule (19) in die Messkammer (11) transportiert wird und dort von den Sensoren (12) die in der Membran (13) gesammelten Gase (4) detektiert werden.

8. Verfahren nach einem der Ansprüche 5 bis 7, wobei
- beim Spülen das Trägergas (16) durch eine erste Leitung (7) angesaugt, durch das Ventil (20) und über die Trennsäule (19) in die Messkammer (11) befördert und durch den Auslass (6) abgeführt wird.

9. Verfahren nach einem der Ansprüche 5 bis 8, wobei
- wobei das Spülen der Messkammer (11) mittels der zweiten Pumpe (10) erfolgt.

10. Verfahren nach einem der Ansprüche 5 bis 9, wobei
- das Befördern des Trägergases (16) mittels einer ersten Pumpe (9) erfolgt.

11. Verfahren nach einem der Ansprüche 5 bis 10, wobei
- die Menge und/oder Art des Gases (4) in der Messkammer (11) bestimmt werden, bevor und/oder nachdem das Trägergas (16) heraus befördert wurde.

## Claims

1. Device (1) for detecting gas (4) in a high-voltage apparatus (3) filled with an insulating medium (2), comprising:
- an inlet (5) for introducing and an outlet (6) for discharging a carrier gas (16);
- at least one gas sensor (12) for detecting a gas (4);
- a first pump (9) for conveying the carrier gas (16) in the device (1);
- a membrane (13) which consists of at least one semi-permeable material, is at least partially surrounded by the insulating medium (2) and is at least partially flowed against by the carrier gas (16);
- a second pump (10) for conveying the carrier gas (16) into the device (1) and for conveying it out of the device (1);
**characterized in that**
- a separating column (19) is provided which is arranged in front of the gas sensor (12) and the gas sensor (12) is designed as a sensor array;
- a valve (20) with at least two operating states;
- a measuring chamber (11) in which sensors for temperature, humidity, pressure and the at least one gas sensor (12) are arranged;
wherein
- the diaphragm (13) is connected on the one hand to a first connection (20.1) via a first connecting line (31) and on the other hand to a second connection (20.2) of the valve (20) via a second connecting line (32);
- the first pump (9) is arranged in the first connecting line (31);
- a fourth connecting line (34) connects the separating column (19) to the measuring chamber (11) via a fourth connection (20.4) of the valve (20);
- the measuring chamber (11) is connected to an outlet (6) via a second line (8);
- a sample loop (21) is connected via a third connecting line (33) to a third connection (20.3) and a fourth connecting line (34) to a fifth connection (20.5) of the valve (20) .

2. Device according to claim 1, wherein
- the diaphragm (13) is at least partially tubular and/or at least partially hose-like.

3. The device according to any one of claims 1 to 2, wherein
- an inlet (5), a first line (7) and a filter (15) are provided and the first line (7) is connected to a sixth connection (20.6) of the valve (20).

4. The device according to any one of claims 1 to 3, wherein
- the sensors in the measuring chamber (11), the first pump (9), the second pump (10) and the valve (20) are connected to a control device (17); and
- the sensors in the measuring chamber (11), the first pump (9), the second pump (10) and the valve (20) are controlled on the basis of the measurements of the sensor in the measuring chamber (11).

5. Method for detecting gas (4) in a high-voltage device (3) filled with an insulating medium (2) by means of a device (1) according to one of the preceding claims, wherein:
- in a first step, a measuring chamber (11) is purged with a carrier gas (16) by conveying the carrier gas (16) through the separation column (19) into the measuring chamber (11) and out of the measuring chamber (11);
- in a second step, the carrier gas (16) is conveyed through a membrane (13) and is enriched by gas (4) flowing through the membrane (13);
- in a third step, a sensor (12) in the measuring chamber (11) measures the gas (4) after it has passed through the separation column (19).

6. The method according to claim 5, wherein
- in the first and third steps, the valve (20) is in a first operating state for purging the measuring chamber (11) and for measuring the gases (4);
- in the third step, the valve (20) is in a second operating state for enriching the carrier gas (16).

7. Method according to claim 5 or 6, wherein
- the carrier gas (16) is transported through a valve (20), a sample loop (21), the fourth connecting line (34) and the separation column (19) into the measuring chamber (11), where the gases (4) collected in the membrane (13) are detected by the sensors (12).

8. The method according to any one of claims 5 to 7, wherein
- during purging, the carrier gas (16) is drawn in through a first line (7), conveyed through the valve (20) and via the separating column (19) into the measuring chamber (11) and discharged through the outlet (6).

9. The method according to any one of claims 5 to 8, wherein
- wherein the measuring chamber (11) is flushed by means of the second pump (10).

10. The method according to any one of claims 5 to 9, wherein
- the carrier gas (16) is conveyed by means of a first pump (9).

11. The method according to any one of claims 5 to 10, wherein
- the quantity and/or type of gas (4) in the measuring chamber (11) is determined before and/or after the carrier gas (16) has been conveyed out.

## Revendications

1. Dispositif (1) de détection de gaz (4) dans un appareil à haute tension (3) rempli d'un milieu isolant (2), comprenant :
- une entrée (5) pour l'introduction et une sortie (6) pour l'évacuation d'un gaz porteur (16) ;
- au moins un capteur de gaz (12) pour la détection d'un gaz (4) ;
- une première pompe (9) pour transporter le gaz porteur (16) dans le dispositif (1) ;
- une membrane (13) qui est constituée d'au moins un matériau semi-perméable, qui est entourée au moins partiellement par le milieu isolant (2) et qui est soumise au moins partiellement à l'écoulement du gaz porteur (16) ;
- une deuxième pompe (10) pour faire entrer le gaz porteur (16) dans le dispositif (1) et pour le faire sortir du dispositif (1) ;
**caractérisé en ce que**
- il est prévu une colonne de séparation (19) qui est disposée devant le capteur de gaz (12) et le capteur de gaz (12) est réalisé sous forme de réseau de capteurs ;
- une vanne (20) ayant au moins deux états de fonctionnement ;
- une chambre de mesure (11) dans laquelle sont disposés des capteurs de température, d'humidité, de pression et le au moins un capteur de gaz (12) ;
dans lequel
- la membrane (13) est reliée d'une part par une première conduite de liaison (31) à un premier raccord (20.1) et d'autre part par une deuxième conduite de liaison (32) à un deuxième raccord (20.2) de la vanne (20) ;
- la première pompe (9) est disposée dans le premier conduit de liaison (31) ;
- une quatrième conduite de liaison (34) relie la colonne de séparation (19) à la chambre de mesure (11) via un quatrième raccord (20.4) de la vanne (20) ;
- la chambre de mesure (11) est reliée à une sortie (6) par une deuxième conduite (8) ;
- une boucle d'échantillonnage (21) est reliée par une troisième ligne de liaison (33) à un troisième raccord (20.3) et une quatrième ligne de liaison (34) à un cinquième raccord (20.5) de la vanne (20) .

2. Dispositif selon la revendication 1, dans lequel
- la membrane (13) est réalisée au moins partiellement en forme de tube et/ou au moins partiellement en forme de tuyau.

3. Dispositif selon l'une quelconque des revendications 1 à 2, dans lequel
- une entrée (5), une première conduite (7) et un filtre (15) sont prévus, et la première conduite (7) est reliée à un sixième raccord (20.6) de la vanne (20).

4. Dispositif selon l'une quelconque des revendications 1 à 3, dans lequel
- les capteurs dans la chambre de mesure (11), la première pompe (9), la deuxième pompe (10) et la vanne (20) sont reliés à un dispositif de commande (17) ; et
- les capteurs dans la chambre de mesure (11), la première pompe (9), la deuxième pompe (10) et la vanne (20) sont commandés sur la base des mesures des capteurs dans la chambre de mesure (11).

5. Procédé de détection de gaz (4) dans un appareil haute tension (3) rempli d'un milieu isolant (2) au moyen d'un dispositif (1) selon l'une des revendications précédentes,
dans lequel :
- dans une première étape, on rince une chambre de mesure (11) avec un gaz porteur (16) en faisant entrer le gaz porteur (16) dans la chambre de mesure (11) et en le faisant sortir de la chambre de mesure (11) à travers la colonne de séparation (19) ;
- dans une deuxième étape, le gaz porteur (16) est transporté à travers une membrane (13) et s'enrichit en gaz (4) qui traverse la membrane (13) ;
- dans une troisième étape, un capteur (12) dans la chambre de mesure (11) mesure le gaz (4) après que celui-ci a traversé la colonne de séparation (19).

6. Procédé selon la revendication 5, dans lequel
- dans les première et troisième étapes, la soupape (20) est dans un premier état de fonctionnement pour purger la chambre de mesure (11) et mesurer les gaz (4) ;
- dans la troisième étape, la vanne (20) est dans un deuxième état de fonctionnement pour enrichir le gaz porteur (16).

7. Procédé selon la revendication 5 ou 6, dans lequel
- le gaz porteur (16) est transporté à travers une soupape (20), une boucle d'échantillonnage (21), la quatrième conduite de liaison (34) et la colonne de séparation (19) dans la chambre de mesure (11) et que les gaz (4) recueillis dans la membrane (13) y sont détectés par les capteurs (12).

8. Procédé selon l'une quelconque des revendications 5 à 7, dans lequel
- lors du rinçage, le gaz porteur (16) est aspiré par une première conduite (7), transporté à travers la soupape (20) et par la colonne de séparation (19) dans la chambre de mesure (11) et évacué par la sortie (6).

9. Procédé selon l'une quelconque des revendications 5 à 8, dans lequel
- le rinçage de la chambre de mesure (11) étant effectué au moyen de la deuxième pompe (10).

10. Procédé selon l'une quelconque des revendications 5 à 9, dans lequel
- le transport du gaz porteur (16) s'effectue au moyen d'une première pompe (9).

11. Procédé selon l'une quelconque des revendications 5 à 10, dans lequel
- la quantité et/ou le type de gaz (4) dans la chambre de mesure (11) sont déterminés avant et/ou après que le gaz porteur (16) a été évacué.
